# EUROPEAN PATENT SPECIFICATION

(11) **EP 3 887 368 B1**
(45) Date of publication and mention of the grant of the patent: **06.12.2023**
(21) Application number: 20903517.9
(22) Date of filing: 07.12.2020
(51) Int. Cl.: C07D 471/00, C07D 471/04, A61P 35/00

(54) **3/4-((2E,6E)-3,7,11-TRIMETHYLDODECA-2,6,10-TRIENYLTHIO)BENZAMIDE DERIVATIVE COMPOUNDS**
3/4-((2E,6E)-3,7,11-TRIMETHYLDODECA-2,6,10-TRIENYLTHIO)BENZAMID-DERIVATVERBINDUNGEN
COMPOSÉS DÉRIVÉS DE 3/4-((2E,6E)-3,7,11-TRIMÉTHYLDODÉCA-2,6,10-TRIÉNYLTHIO)BENZAMIDE

(30) Priority: 20.12.2019 TR 201920922
(43) Date of publication of application: 06.10.2021
(73) Proprietor: Ankara Üniversitesi Rektörlügü, 06100 Ankara (TR)
(72) Inventor: BÜYÜKBINGÖL, Mehmet Erdem, Ankara (TR); DEMIRCI TÜRE, Asli, Ankara (TR); ÖZPOLAT, Bülent, Ankara (TR)
(74) Representative: Kaya, Erdem
(86) International application number: PCT/TR2020/051245
(87) International publication number: WO 2021/126120

(56) References cited:
- WO-A1-2013/052765
- WO-A1-2013/171640
- WO-A2-2011/090738
- CN-A- 103 113 274
- US-A1- 2016 207 891
- US-B2- 10 011 600

## Description

### Technical Field of the Invention

The present invention relates to benzamide derivative compounds that are potential RAS (Kirsten Rat Sarcoma Virus) inhibitors and/or potential farnesyltransferase (FTase) inhibitors.

### State of the Art

Cancer, compared to various other diseases, is a health problem, of which treatment requires greater effort. In addition, the population of cancer patients grows increasingly all around the world. It is anticipated that the number of cancer patients in 2020 will double the number of cancer patients in 2010. Moreover, the treatment of cancer, unfortunately, fails in most cases. Quite naturally, this particular situation serves as an impetus for scientific circles to exert greater efforts in the treatment of tumors. Even though all mechanisms triggering cancer have yet to become clear, it is known for a fact that there are several mechanisms and pathways that affect the development of cancer. Such pathways are scrutinized, and new options are constantly developed for the treatment of cancer.

In cancer, many important cellular functions including cellular growth, differentiation, and survival occur differently from regular functioning. RAS protein, performing various tasks in signaling pathways of cells, assumes a crucial role in regulating vital functions such as DNA synthesis, cellular proliferation, differentiation, and migration. The mutation of RAS protein, which is one of the key proteins for cells, serves as an intermediary in cancer development by overstimulating mitogenic functions. Mutated RAS proteins are the reason for 30% of human tumors. The prevalence of RAS mutation is 90% in pancreatic cancer, 45% in colorectal carcinoma, 35% in lung cancer, and 10% in bladder cancer. Considering the cancer types induced by RAS mutation, pancreatic cancer takes 5^{th} place in Europe, and 4^{th} place in America when it comes to causes of death. Therefore, RAS inhibition is an important field of study for scientific communities. Inhibition of RAS protein is a promising approach in the treatment of various tumors. One of the studies that aim to inhibit the activity of RAS protein led to the development of FTS (S-trans, trans-farnesyl thiosalicylic acid; Salirasib) compound. FTS inhibits RAS protein from binding to the cell membrane. Thus, RAS protein cannot stimulate signaling pathways in the cell as it cannot bind to the cell membrane. As reported in the relevant literature, various studies have proven the high efficacy of FTS compound in the inhibition of mutated RAS protein, and there are ongoing studies in different phases for other cancer varietals. Studies conducted in this particular subject have demonstrated that amide derivatives of FTS compound feature even higher efficacy in tumor inhibition. Novel drugs for various diseases are designed with drug candidate molecules by the disciplines working on designing, synthesizing, and determining the biological activity of new drugs. However, respective searches conducted in the relevant literature in Turkey yield no results with respect to drug design studies and/or molecules that may be deemed as drug candidates targeting the KRAS protein activity. The present invention that aims to inhibit the activity of KRAS protein is a novel, original study an equivalent of which has yet to be conducted in the world.

Several patent documents have been identified as a result of the search conducted in the relevant art, however, none of these documents bears similarities with the present invention.

The patent document numbered US9562019B2 discloses a method for inhibiting the proliferation of a tumor cell that carries or expresses a cancer-associated epidermal growth factor receptor (eGFR) and/or Kirsten rat sarcoma viral oncogene homolog (KRAS) mutation.

The patent application numbered CR20160205A relates to compounds with activity as inhibitors of G12C mutation of KRAS protein.

The patent document numbered WO2016172187A1 relates to the use of peptides for inhibiting KRAS/Raf interaction.

The patent application numbered JP2018511631 discloses compounds with activity as inhibitors of G12C mutation of KRAS protein.

### Technical Problems the Invention Seeks to Solve

RAS proteins are guanine nucleotide-binding proteins responsible for various cellular functions including cell proliferation, differentiation, migration, as well as apoptosis. RAS proteins feature 3 different types which are NRAS, HRAS, and KRAS. These proteins bear resemblance to one another at a rate of 90%. RAS proteins activate *downstream* effectors containing MAPK, PI3K, RALGDS, and PLC pathways after the cell is stimulated by various growth factors. Thus, the RAS protein ensures that growth and differentiation are regulated and under control. When a mutation occurs in RAS genes encoding RAS proteins, these cells transform into cancer cells, thereby resulting in the development of various cancer types. Several studies aiming at RAS protein are conducted in order to treat the cancer types associated with RAS protein activation. Respective studies carried out to that end have led to the development of the FTS compound, and this compound's efficacy in the inhibition of RAS activity by preventing the RAS protein from binding to cell membrane, and the anticancer effects thereof on several cancer types have been demonstrated via a number of studies. In addition thereto, amide derivatives of FTS have proven to feature even higher activity on tumor inhibition. Based on the anticancer effects of the FTS compound, the present invention comprises the synthesis of 14 substances, 12 of which are entirely original. Initially, the phenyl ring in the FTS compound and the farnesylthio- group attached to the ring were preserved, and carboxylic acid-bound compounds (3-FTB, 4-FTB) were obtained from the meta and para positions based on the farnesylthio- group attached to the phenyl ring. Subsequently, non-substituted amide (3-FTBA, 4-FTBA) and substituted amide derivatives (3 (a, b, c, e, f), 4 (a, b, c, e, f)) were developed over the 3-FTB and 4-FTB compounds. Synthesized compounds were purified via column chromatography by using suitable solvent systems. Purities of compounds were checked via TLC and melting points of the ones in solid form were determined. Accordingly, expected chemical structures of compounds were substantiated through the results of elemental analysis, mass (ESI), as well as ¹H-NMR spectral results. The authenticity of all compounds was checked by using chemical databases.

### Description of the Invention

Twelve original compounds in the present invention were created in vitro for the first time, and the structures of compounds were clarified through various instrumental analysis methods. These compounds are; 3-((2*E*,6*E*)-3,7,11-trimethyldodeca-2,6,10-trienylthio)benzamide, 4-((2*E*,*6E*)-3,7,11-trimethyldodeca-2,6,10-trienylthio)benzamide, *N-*(2-(1*H*-indole-3-yl)ethyl)-3-((2*E*,6*E*)-3,7,11-trimethyldodeca-2,6,10-trienylthio)benzamide, *N*-(2-(5-methoxy-1*H*-indole-3-yl)ethyl)-3-((*2E*,*6E*)-3,7,11-trimethyldodeca-2,6, 10-trienylthio)benzamide, *N*-(2,2,6,6-tetramethylpiperidine-1-oxyl-4-yl)-3-((2*E*,6*E*)-3,7,11-trimethyldodeca-2,6,10-trienylthio)benzamide, *N*-(1*H*-benzo[d]imidazole-2-yl)-3-((*2E*,*6E*)-3,7,11-trimethyldodeca-2,6,10-trienylthio)benzamide, *N*-(benzo[d]thiazole-2-yl)-3-((2E,6E)-3,7,11-trimethyldodeca-2,6,10-trienylthio)benzamide, *N*-(2-(1H-indole-3-yl)ethyl)-4-((2*E*,6*E*)-3,7,11-trimethyldodeca-2,6,10-trienylthio)benzamide, *N*-(2-(5-methoxy-1*H*-indole-3-yl)ethyl)-4-((2*E*,6*E*)-3,7,11-trimethyldodeca-2,6,10-trienylthio)benzamide, *N*-(2,2,6,6-tetramethylpiperidine-1-oxyl-4-yl)-4-((2*E*,*6E*)-3,7,11-trimethyldodeca-2,6,10-trienylthio)benzamide, *N*-(1*H*-benzo[d]imidazolc-2-yl)-4-((*2E*,*6E*)-3,7,11-trimethyldodeca-2,6,10 trienylthio)benzamide, *N*-(benzo[d]thiazole-2-yl)-4-((2E,6E)-3,7,1 1-trimethyldodeca-2,6,10-trienylthio)benzamide.

Twelve original compounds of the present invention may be used in the treatment of cancer or other diseases. The inventive 3/4-((2*E*,6*E*)-3,7,11-trimethyldodeca-2,6,10-trienylthio)benzamide derivative compounds are potential RAS inhibitor and/or potential farnesyltransferase inhibitor compounds, and the aforementioned 12 original compounds bear the characteristics of drug candidate compounds. Biological activity tests were performed on the inventive compounds, and these compounds were tested on various types of cancer cells and protein systems.

### Detailed Description of the Invention

RAS protein is a mutated protein in many different types of cancer and features expressions at many different levels. RAS protein is a guanine nucleotide-binding GTPase (Guanine Nucleotide Triphosphatase) ensuring the developmental and proliferative information transfer from the extracellular region to the cell nucleus. RAS protein serves as a molecular key responsible for regulating many cellular pathways including cellular proliferation, differentiation, and apoptosis.

There are three different types of RAS protein. These proteins are encoded by RAS genes. Three protein-encoding proto-oncogenes have been identified, these proto-oncogenes are the Harvey-RAS gene, Kirsten-RAS gene, and Neuroblastoma-RAS gene. These genes encode RAS proteins (HRAS, NRAS, KRAS 4A, and KRAS 4B) that are referred to as p21 and have a molecular weight of 21 kilodaltons (kDa). RAS proteins are fundamentally isoforms of one another and quite similar in terms of functions. RAS proteins consist of three domains: A domain of 85 amino acids identical in all three forms, a domain of 86-165 amino acids bearing 90% similarity in three forms, and the carboxy-terminal (CAAX) domain between the amino acid residue of 165-188/189 that separates three forms from one another. Although not completely identical, the amino acid sequence between the 1^{st} and 165^{th} amino acids is significantly similar in HRAS, NRAS, and KRAS. Isoforms are separated from one another by the last 23/24 amino acid. The separated region is called the hypervariable region (HVR). Hypervariable regions (HRV) bear 10-15% resemblance with one another. These hypervariable regions (HVR) are responsible for the functions involving the attachment of RAS protein to the cell membrane.

### Posttranslational Modifications (PTM) of RAS Protein

RAS protein is synthesized as a biologically inactive precursor protein and available in the cellular cytoplasm. It is imperative for the RAS protein to communicate with the cell membrane to be able to generate a signal within the cell. RAS protein undergoes various changes from the carboxy-terminal before transforming into an active protein state. These changes increase the hydrophobicity of the protein, thereby enabling it to fit into the inner surface of the cell membrane. The protein cannot gain the characteristic of attaching to the cell membrane without undergoing posttranslational modification.

RAS protein is a type of protein that contains CAAX residue. In CAAX, "C" represents the cysteine residue, "A" represents the aliphatic amino acid residue, and "X" represents any amino acid. The protein undergoes various changes from the CAAX residue to be able to communicate with the cell membrane. The entirety of these changes which increase the hydrophobicity of RAS protein is called posttranslational modification. Posttranslational modification (PTM) modifies the last 20 amino acids of the protein, thereby increasing both the complexity and function thereof. RAS protein gains the characteristic of being able to attach to the cell membrane following the respective modifications. Posttranslational modification of RAS protein comprises the following steps:
- Prenylation Stage
- Proteolysis and Carboxy-methylation Stage
- Palmitoylation Stage

### Prenylation Stage

Prenylation is the initial stage of posttranslational modification. A lipid chain is attached to the RAS protein during the prenylation stage. During this step, a farnesyl chain is transferred to the cysteine in the CAAX terminal of RAS protein. Catalysis of the farnesyl chain with 15 carbons from the farnesyl pyrophosphate (FPP) molecule is performed by the farnesyltransferase (FTase) enzyme. RAS protein gains the capability of binding to the endoplasmic reticulum (ER) membrane following the farnesylation. Farnesyltransferase enzyme inhibitors (FTI) inhibit the farnesylation of RAS protein, in other words, FTI inhibits RAS protein from binding to the ER. As the RAS protein, the farnesyl group of which does not bond remains in the cytoplasm, it cannot stimulate activation pathways. The prenylation is stage is a rate-controlling step for the posttranslational modification.

As an alternative to farnesylation, the 20-carbon geranylgeranyl group may be transferred to RAS protein in the prenylation step by means of the catalysis of geranylgeranyl transferase enzyme over the geranylgeranyl pyrophosphate.

### Proteolysis and Carboxy-Methylation Stage

Subsequent to the prenylation stage, RAS protein gets separated from the AAX residue of the CAAX terminal in ER. The process of proteolysis is performed by catalyzing the RAS converting enzyme 1 (RCE1) which is a protease. Subsequently, RAS undergoes methyl esterification in the ER through yet another enzyme catalyst. This enzyme is isoprenylcysteine carboxy-methyltransferase (ICMT), and it ensures the methyl esterification of α-carboxyl of ICMT farnesyl cysteine. It should be noted that only the step catalyzed by ICMT is cyclic among the aforementioned steps.

### Palmitoylation Stage

In palmitoylation, which is the final step of posttranslational modification, the 16-carbon palmitoyl fatty acid group is transferred to the terminal cysteine residue of protein over the palmitoyl coenzyme A. This transfer is a transfer of the acyl group.

HRAS contains two cysteine residues which are 181^{st} and 184^{th} amino acids and undergoes palmitoylation therefrom. NRAS undergoes palmitoylation from the cysteine which is the 181^{st} amino acid thereof. While KRAS 4B does not undergo palmitoylation, KRAS 4A undergoes palmitoylation from cysteine which is the 180^{th} amino acid thereof. KRAS 4B is capable of bonding to the cell membrane without undergoing palmitoylation due to its polybasic region. Palmitate forms a variable thioester bond with RAS. The modification that realizes upon adding palmitate is reversible.

Consequently, RAS protein undergoes prenylation, proteolysis, carboxy-methylation, and palmitoylation stages by means of posttranslational modification, thereby increasing its hydrophobicity. Subsequently, the localization of the protein to the cell membrane is carried out. RAS protein, being bonded with the cell membrane, gains the capability of responding to extracellular stimuli.

### Activation-Inactivation Balance of RAS Protein

Activation of the RAS protein depends on whether it is GTP-bound (guanosine triphosphate)) or GDP-bound (guanosine diphosphate. When the RAS protein is GTP-bound, the protein is active and capable of associating with *downstream* target enzymes. In contrast, when the RAS protein is GDP-bound, the protein is inactive and cannot associate with effectors. The activity of RAS proteins in normal and non-mutated cells is controlled by the ratio of protein-bound GTP amount to protein-bound GDP amount. Processes of GDP-GPT binding to RAS protein and exchange thereof is carried out through the catalysis of guanine exchange factors (GEFs) and GTPase activating proteins (GAPs). Guanine exchange factors (GEFs) are responsible for the nucleotide exchange and ensure the activation of the RAS protein. GTPase activating proteins (GAPs), on the other hand, are responsible for the nucleotide hydrolysis and ensure the inactivation of RAS protein. Large protein families participate in performing these two activities in order to ensure the activation and inactivation of RAS protein. Although there are various GEFs and GAPs within a cell, guanine nucleotide releasing factor 1 (GRF1) may be one of the many examples of GEFs. RAS-GRF1 ensures the activation of RAS protein, and accordingly, RAS-associated *downstream* effector pathways are stimulated.

### RAS Protein Activation

The necessary guanine nucleotides are released for the activation of RAS even if a cell is not stimulated by any growth factor. However, the pace of this release process is slow. When a cell is stimulated by a stimulant, the release of guanine nucleotides is accelerated by GEFs. The catalytical mechanism herein comprises a sequence of fast reactions transforming from the RAS-nucleotide complex and RAS-nucleotide-GEF complex into a complex without nucleotides. These reactions are cyclic.

One must understand the structure of a protein to be able to comprehend the activation-inactivation balance of the RAS protein. RAS protein consists of 6 β-sheets and 5 α-helices. These structures are bound by another with 10 loops (loop-L). Five of these loops settle to the outer surface of the protein and assume a vital task in regulating RAS nucleotide interactions. GTP y-phosphate interacts with 1st, 2nd, and 4th loops (L1, L2, L4) and settles to RAS (e.g. Liz16, Tir32, Tir35, Gli60, and Glu61). There is a difference between the GTP-bound and GDP-bound conformation of RAS protein. Said difference can be observed in the *switch I* and *switch II* regions. *The switch I* correspond to the region between 32 and 38 and contains the range between L2 and β2. *Switch II,* on the other hand, corresponds to the region between 59 and 67. The *Switch II* portion is in motion and available in various conformations.

When the cellular surface is stimulated by the effector protein, GEFs interact with *Switch I* and *Switch II* regions of RAS protein and ensure the GTP bond between these regions. The GTP bond changes the structure of *switch I.* This change stems from the alteration that occurs in the Tir35 chain. Hence, GTP y-phosphate can interact with the Mg²⁺ion. Analogously, y-phosphate interacts with the Gli60 chain and a change occurs in the *Switch II* region.

The process that begins with the stimulation of the cellular surface by an effector and reaches the step of conversion of the RAS protein to the GTP-bound active state thereof will be described in detail below. When a growth factor such as FGF, PDGF, NGF, EGF, or insulin reaches the cell, the tyrosine kinase receptor is stimulated by this growth factor. Stimulated tyrosine kinase receptor goes under autophosphorylation and dimerization, and subsequently, interacts with SHC. The activated receptor binds to the GRB2 adaptor protein. SOS protein undergoes phosphorylation thereafter and becomes active. SOS stimulates GEFs so that they can reach the plasma membrane. Once GEFs reach the cell membrane, they ensure the RAS-GDP RAS-GTP exchange and activate the RAS protein.

### RAS Protein Inactivation

RAS protein inactivation is based on the hydrolysis of GTP to GDP. While the L9 and L10 loops in the structure of the RAS protein interact with the guanine base, L1, L2, and L4 loops create an active region around the y-phosphate group. L1 is referred to as the phosphate-binding loop, while L2 and L4 loops serve as a target for GAPs. A loop of 32-40 amino acids is defined as an effector loop and contains the L2 and β-2 chain. This loop undergoes structural changes when the y-phosphate group gets separated. As disclosed in the RAS activation section, one of the most significant differences between GTP-bound and GDP-bound forms of RAS is the region in which the catalytic activity is observed, namely, the region where the hydrogen bonds between y-phosphate and L2, L4 break. In this region, the hydrogen bond between the Tir35 main chain and y-phosphate breaks. In addition thereto, it separates from the Mg²⁺ ion of -OH group located in the side chain and remains in the medium. These changes occurring in the Tir35 affect the other neighboring amino acid residue and cause some particular amino acids that are directly exposed to water to move towards the surface of the protein. On the other hand, in the region of 60-76 amino acids (L4 and α-2), a conformational change occurs in L4 upon the break of the hydrogen bond between the Gli60 and y-phosphate group. As a result of the hydrolysis, GTP form transforms into GDP form, and additionally, a change occurs in the protein conformation.

### Relationship Between the Mg²⁺ Ion and RAS Protein

Proteins transferring phosphoryl group either to or from nucleotide generally associate with these nucleotides via the Mg²⁺ complexes they contain. This enzymatic process depends on the availability of divalent metal ions. Mg²⁺ creates a shield against the attacks to negative charges in the y-phosphate group and increases the acid strength of the β-phosphate group to be separated, thereby activating nucleophilicity. The affinity of guanine nucleotides to protein increases in the presence of a divalent metal ion and the rate of dissociation decreases. For RAS protein, this particular condition follows the course stated below; RAS's affinity to GTP or to GDP increases by 500 times in the presence of Mg²⁺.

It is demonstrated that the Mg²⁺ ion is positioned through an octahedral arrangement with six oxygen atoms in the 3-dimensional structure of the RAS protein. Mg²⁺ion bonds with phosphate oxygens of two guanine pyrophosphate. Additionally, Mg²⁺ establishes bonds with Ser17 and Tir35 amino acids of the RAS protein. The remaining two bonds of the octahedral structure of Mg²⁺ion are completed through the bonds established with water molecules. Asp57 amino acid also plays a role in the settling of Mg²⁺' to the RAS protein.

### Downstream and Upstream Processes of RAS Protein

RAS proteins stimulate the synthesis of growth factors in the cell. Synthesized growth factors, along with other functions in the body, bind to certain receptors like EGFR (Epidermal Growth Factor Receptor) located in cells in which it is synthesized due to autocrine effects, and creates a mitogenic response in the cell. This particular sequence of processes is called the *upstream* process. The *upstream* process results in cellular proliferation. The reason why the *"Upstream* process" is given this particular name is that some growth factors synthesized in association with the RAS protein go out of the cell and produce effects by bonding to corresponding receptors on the cellular surface.

The *downstream* process, on the other hand, comprises the activation of the RAS protein by the bonding of growth factors, which are available within the organism in a synthesized state through the RAS-bound pathway, to their respective receptors, and correspondingly, the stimulation of signaling pathways by the RAS protein. This process is called the *"Downstream* process" because of the fact that is carried out via an extracellular stimulant, namely, the growth factors, and the RAS protein is activated as a result thereof.

### Signaling Pathways of RAS Protein

RAS protein maintains control over various cellular functions such as cell proliferation and differentiation via the pathway affected thereby. When the RAS protein gets activated upon becoming GTP-bound, it initiates intracellular catalytic activity by communication with various effector protein families.

The first pathway identified in RAS effector signaling is the mitogen-active protein kinase (MAPK) pathway which is also known as the Raf-MEK-ERK signal transduction pathway. There are three types of RAF proteins which are A-Raf, B-Raf, C-Raf/Rafl. Raf serine/threonine kinases communicate with GTP-bound RAS and ensure the Raf protein kinase activation. Raf activates MEK by phosphorylating MEK ERK following the activation of the RAS protein. ERK takes an important place in the regulation of the cell cycle since it comprises several substrates within the cell cytoplasm and nucleus. Events related to the cell cycle and cellular proliferation are activated through the sequence of processes disclosed above.

Phosphoinositole-3-kinase (PI3K) pathway is yet another pathway affected by the RAS protein. PI3K catalyzes the conversion of phosphatidylinositol-4,5-biphosphate (PIP₂) to phosphatidylinositol-3,4,5-triphosphate (PIP₃). PI3K is responsible for the translocation and conformational changes to the cell membrane. P3IK gets activated depending on the stimulation of the tyrosine kinase receptor independently of the RAS protein, however, the basic principle in P3IK activity is that P3IK gets activated after the activation of the RAS protein through GTP. PI3K signaling relates to cellular processes that may be significant in oncogenic transformation and it activates the AKT/PKB and PDK1 proteins which are of vital importance. AKT/PKB protein features a quite potent anti-apoptotic function. It performs this function by ensuring the inactivation of BAD and Forkhead apoptotic proteins. Thus, the survival of the cell is ensured. Consequently, through the P3IK pathway, RAS protein performs the processes of controlling the cytoskeleton, cellular movement, and the prevention of cellular aging. In addition thereto, RAS-GTP binds directly to the protein (TIAM1) inducing spreading and metastasis of tumor, thereby supporting the development of tumors.

RALGDS proteins are the third pathway affected by the RAS protein. These are the guanine nucleotide exchange factors for Ral which is a RAS-associated protein. Ral proteins, just like RAS proteins, are inactive when they are GDP-bound, and active when they are GTP-bound. Activated Ral proteins regulate the circulatory functions of the cytoskeleton and vessels.

Finally, phospholipase C (PLC) is yet another pathway affected by the RAS protein, and PLC catalyzes the hydrolysis of cellular membrane lipids. RAS catalysis ensures the conversion of phosphatidylinositol-4,5 to diacylglycerol and inositol-1,4,5-triphosphate in the cellular membrane. Thus, Ca²⁺ is released and protein kinase C activation is initiated through the Ca²⁺-mediated signaling.

### Role of the RAS Protein in Cancer

The role assumed by the RAS superfamily in cancer has been fully clarified. RAS protein may display oncogenic activity under some extraordinary conditions. The mechanisms through which the RAS protein causes cancer are described below. The first of these mechanisms is the overstimulation of receptors located on the cell membrane, and correspondingly, the overactivity of the RAS protein due to the synthesis of an excessive amount of growth factor in the *upstream* process. Another mechanism that triggers the RAS-associated cancer is the fact that the RAS protein constantly maintains an active state because of being unable to become separated from the GTP complex due to the mutation-induced activation of the RAS protein.

### Mutation Induced Activation of the RAS Protein

The mutation of the RAS gene encoding the RAS protein is observed in approximately 30% of the cancerous cells. The mutation usually occurs in the 12^{th}, 13^{th}, 59^{th}, and 61^{st} codons of the RAS gene. However, the most frequently observed mutation occurs in the 12^{th} codon. The mutation in the 12th codon causes glycine to convert into serine, cysteine, arginine, asparagine, alanine, or valine. Bladder cancer caused by the mutation of the 12^{th} codon, myeloid leukemia caused by the mutation of the 13^{th} codon, leukemia caused by the mutation of the 59^{th} codon, and lung cancer caused by the mutation of the 61^{st} codon are among the examples of cancers induced by the RAS mutations.

Gene mutations cause abnormal RAS signaling in cancerous cells. KRAS mutation, with a rate of 85% is the mutation with the highest prevalence among the RAS mutations. KRAS mutation is followed by NRAS mutation with approximately 15%, and HRAS mutation with a rate lower than 1% respectively. The clinical significance of these mutations caused by the differences in isoforms has yet to be clarified. However, it is known that RAS isoforms produce different biochemical results based on the fact that they activate different *downstream* pathways.

All of these mutations induce changes in the GTPase activity. GTP hydrolysis performed by GAPs is inhibited in case there is a mutation in the gene. Thus, RAS protein remains GTP-bound and maintains a constant active state.

The overstimulation of the Raf pathway correspondingly causes the overstimulation of the production of angiogenic factors and matrix metalloproteinases over the ERK proteins. Hence, the factors required for cellular proliferation and differentiation are created, thereby giving rise to the development of a tumor. B-Raf has been determined to be overactive in most of the cancerous cells, and particularly in melanoma (~ %70) and colon cancer (~ %15). Overstimulation of the growth factor receptor, namely tyrosine kinase, results in activation of the RAS signaling pathway. For instance, EGFR and ERBB2 are overstimulated in breast and ovary cancers, and the AKT2 activity increasing due to the overstimulation of the PI3K pathway causes tumorigenesis. Stimulation of AKT2 results in loss of susceptibility to apoptosis. This forces the cell to proliferate. However, the main reason behind tumors that develop due to the overstimulation of the PI3K pathway is the deletion of PTEN (phosphate and tension homolog), namely, the tumor suppressor gene. The PTEN gene is deleted in 30-40% of tumors, and it takes the second place in terms of importance after TP53, as the most important tumor suppressor gene, encoding the p53 protein.

### RAS Protein as a Target in Cancer Therapy

RAS protein is active when it is GTP-bound, and inactive when GDP-bound. The activation-inactivation balance of RAS is ensured by the GEFs and GAPs. Oncogenic mutation of the RAS gene blocks functions of GAPs which accelerate the hydrolysis of GTP. Therefore, the most direct approach among various anticancer therapies targeting the RAS protein is eliminating this blockage. However, academic studies conducted in this regard has yet to yield any successful results. Instead, different therapy methods are being investigated.

### Inhibiting RAS Protein from Binding to Cell Membrane

RAS is available in the cytosol after being synthesized within the cell. RAS needs to bind to the cell membrane to be able to produce effects. An intervention to the binding process of the mutated RAS to the cell membrane may potentially inhibit the effects of the mutant RAS on the cell.

### Farnesyltransferase Inhibitors (FTI) and Geranylgeranyl Transferase Inhibitors (GGTI)

To be able to show activity within a cell, RAS protein needs to bind to the cell membrane and undergo a sequence of posttranslational modifications from the carboxy-terminal. The processes that realize from the CAAX terminal of the protein are also necessary for the RAS protein to convert into an oncogenic state. The inhibition of the posttranslational modification among other anticancer therapy approaches targeting the RAS protein is an alternative anticancer therapy approach investigated by the circles working on drug design.

Adding the farnesyl group to the carboxy-terminal of the protein is the first and foremost of the posttranslational modifications. Adding this lipid group to the RAS protein is carried out by the farnesyl transferase enzyme. Various clinical studies have been conducted on FTase inhibitor anticancer agents; accordingly, oral and *in vitro* administrations thereof have been proven to be tolerable by the body.

The clarification of the crystalline structure of the farnesyl transferase enzyme further facilitated the studies on farnesyl transferase inhibitors. The farnesyltransferase enzyme consists of α subunit of 48 kDa, β subunit of 48 kDa, and aromatic pocket. The pocket comprises two clefts: The first of these clefts is the one to which the CAAX end of the RAS protein binds, and the other is the one to which the farnesyl pyrophosphate group binds. The pocket also contains Zn²⁺ ions. Zn²⁺ is located between the hydrophilic surface of the α subunit and aromatic portions of the β subunit, wherein Zn²⁺ has a tetrahedral geometry and is positioned inside the hydrophobic pockets. Zn²⁺ ion is essential for catalyzation and responsible for activating the nucleophilic attack of farnesyl pyrophosphate towards the cysteine thiol at the CAAX end. Once the farnesyl pyrophosphate has settled to the enzyme, the isoprenoid part gets positioned inside the hydrophobic cleft, while the diphosphate group gets positioned such that it is adjacent to the Zn²⁺ ion.

Three basic approaches have been proposed for inhibiting the farnesyltransferase enzyme:
- Synthesis of FDP analogs for preventing FDP from binding to FTase
- Synthesis of peptidomimetics for preventing the CAAS part of RAS from binding to FTase
- Synthesis of bisubstrate analogs carrying both FDP analogs and peptidomimetics

In addition, non-peptidomimetic FTase inhibitors have been synthesized. However, non-peptidomimetic FTase inhibitors made significant progress in studies conducted on the inhibition of the RAS protein.

Tipifarnib is the first FTase inhibitor tested clinically. Several studies have been conducted on the compound, and phase I, phase II, and phase III clinical trials are still ongoing for many different types of cancer. Phase I trials of Lonafarnib compound, which is another FTase inhibitor, was commenced in 1997. Phase I, phase II, and phase III clinical trials of this compound are still ongoing for different types of cancer. Clinical studies conducted on the BMS-214622 compound, which is an FTI, and on the L-778123 compound which is a dual prenyl transferase inhibitor (DPTI) are significantly less compared to other varietals. The combined administration of BMS-214622 and L-778123 compounds has proven to induce apoptosis in leukemia. Clinical investigations on these two compounds are still ongoing.

Just as the farnesyl transferase enzyme can be inhibited in the prenylation stage, the geranylgeranyltransferase enzyme may also be inhibited during this stage. However, the number of proteins that get activated by binding geranylgeranyl is far greater than the number of proteins that become activated by binding farnesyl. Therefore, the use of geranylgeranyltransferase inhibitors results in extensive toxicity.

### Post-Prenylation Inhibitors

The Post-prenylation step consists of the proteolysis stage and carboxy-methylation stage. RAS protein is separated from the AAX residue of the CAAX terminal in the ER through the catalysis of *RAS* converting enzyme 1 (RCE1) following prenylation. Subsequently, it undergoes methyl esterification by isoprenylcysteine carboxyl methyltransferase (ICMT).

Other pharmacological inhibitors targeting the RAS pathway inhibit RCE1 and ICMT enzymes. Although the data on the importance of the inhibition of the proteolytic cleavage stage with respect to the inhibition of RAS's function was insufficient till the early 1990s, new studies conducted in this regard revealed that RAS conversion fails in the absence of RCE1. Nevertheless, studies conducted on ICMTI and RCElI remain uncertain.

### FTS's Role and Importance in Inhibiting RAS Activity

FTS compound is the product of the studies targeting the RAS protein and a farnesyl cysteine mimetic that prevents the RAS protein from interacting with the cell membrane. FTS inhibits the posttranslational modification of the RAS protein. Respective *in vitro* studies have proven that FTS inhibits tumor growth. Several preclinical studies conducted in this regard have demonstrated that FTS has effects on various cancer types. These tumor types include breast cancer, colorectal carcinoma, glioblastoma, malignant neurofibromatosis type 1, Merkel cell carcinoma, ovary carcinoma, prostate cancer, and pancreatic cancer (Bustinza-Linares et al., 2010). In addition, there are still ongoing clinical trials on the FTS compound, and the *in vivo* efficacy thereof is still being tested. FTS compound has completed phase III for the treatment of pancreatic cancer, phase II for multinodular goiter and lung cancer, and phase I for leukemia.

The chemical structure of the FTS compound (S-trans,trans-farnesyl thiosalicylic acid; Salirasib) is shown below.

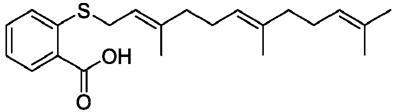

In a clinical study, the antitumorigenic effects of FTS were tested on solid tumors and lymphoma (except for multiple myeloma). FTS was reported to extend the patients' lifespan between 4 to 23 months, and accordingly, FTS's use in phase II was approved.

Phase II clinical trials on FTS's use in the treatment of pancreatic cancer reported that FTS's administration through oral route in combination with gemcitabine has increased the lifespan approximately by 4.7 months.

### Development of Novel Drug Candidate 3/4-((2E,6E)-3,7,11-Trimethyldodeca-2,6,10-trienylthio)benzamide Derivative Compounds

### Methods Implemented in the Analyses of Developed Substances

### Chromatographic Analyses

Thin-Layer Chromatography (TLC) was utilized during the synthesis studies in order to monitor the progress of reactions and to determine the purity degrees of obtained products. To that end, Kieselgel-60 GF₂₅₄-coated aluminum plates *(Merck)* were used. A UV Light operating at 254 nm wavelength (Camag UV Light) was used for identifying stains. Silicagel 60 (0.040-0.060 mm; 230-400 mesh) was used for column chromatography.

### Determination of Melting Points

Melting points were determined via Electrothermal 9100 through the capillary method and results are given without being rectified.

### Elemental Analysis

Elemental analyses were carried out by using the LECO CHNS-932 elemental analysis device.

### Spectral Analyses

### Mass Spectrums

Masses were analyzed via Waters ZQ Micromass LC-MS spectrometer through electrospray ionization (ESI) method.

### Nuclear Magnetic Resonance (NMR) Spectrums

¹H-NMR spectra were obtained in CDCl₃ (Acros) by using a Varian Mercury-400 FT-NMR spectrometer.

### Chemical Substances Used in Synthesis Steps

*Trans,trans*-farnesylbromide (*Aldrich*), 3-Mercaptobenzoicacid (*Aldrich*), 4-Mercaptobenzoicacid (*Aldrich*), Guanidine carbonate (*Aldrich*), 2-(1*H*-Indole-3-yl)ethanamine (tryptamine) (*Aldrich*), 2-(5-Methoxy-1*H*-indole-3-yl)ethanamine (5-methoxytryptamine) (*Aldrich*), 4-Amino-2,2,6,6-tetramethylpiperidine-1-oxyl (4-amino-TEMPO; Tempamine) (*Aldrich*), 2-Aminobenzimidazole (*Aldrich*), 2-Aminobenzothiazole (*Aldrich*), Hydrochloric acid (37%) (*Riedel*-*deHaën*), 1-Ethyl-3-(dimethylaminopropyl)carbodiimide hydrochloride (*Aldrich*), 1-Hydroxybenzotriazole (HOBt) (*Aldrich*)*,* Dimethylformamide (DMF) *(Merck),* Triethylamine (TEA) (*Aldrich*), Benzotriazole-1-yl-oxy-tris-(dimethylamino)phosphonium hexafluorophosphate (BOP) (*Aldrich*), Silicagel 60 (0,040-0,060 mm; 230-400 mesh) *(Merck),* Citric acid *(Merck),* Magnesium sulfate (*Sigma*-*Aldrich*), Sodium bicarbonate *(Merck),* Ammonium chloride *(Merck),* Acetone (*Riedel*-*deHaën*), Chloroform (*Riedel*-*deHaën*), Dichloromethane (*Riedel*-*deHaën*), Ethyl acetate (*Riedel*-*deHaën*), n-Hexane *(Merck).*

### Synthesis of Designed Derivatives

### General Synthesis Method for 3-Farnesylthiobenzoic Acid (3-FTB) and 4-Farnesylthiobenzoic Acid (4-FTB)

3-Mercaptobenzoic acid (6 mmol) / 4-mercaptobenzoic acid (6 mmol), *trans,trans-*farnesylbromide (6 mmol), and guanidine carbonate (16.6 mmol) were mixed within 50 ml of acetone overnight at room temperature. Acetone was concentrated and chloroform was added along with a few drops of 2N HCl. The mixture was washed with distilled water. The organic phase was separated and dried with magnesium sulfate and concentrated. The raw product was purified by using a dichloromethane/ethyl acetate (5:1-2:1) solvent system via column chromatography. Synthesis of 3-trans,trans-farnesylthiobenzamide derivatives (3-FTBA, 3 (a, b, c, e, f)) and 4-trans,trans-farnesylthiobenzamide derivatives (4-FTBA, 4 (a, b, c, e, f) is shown below.

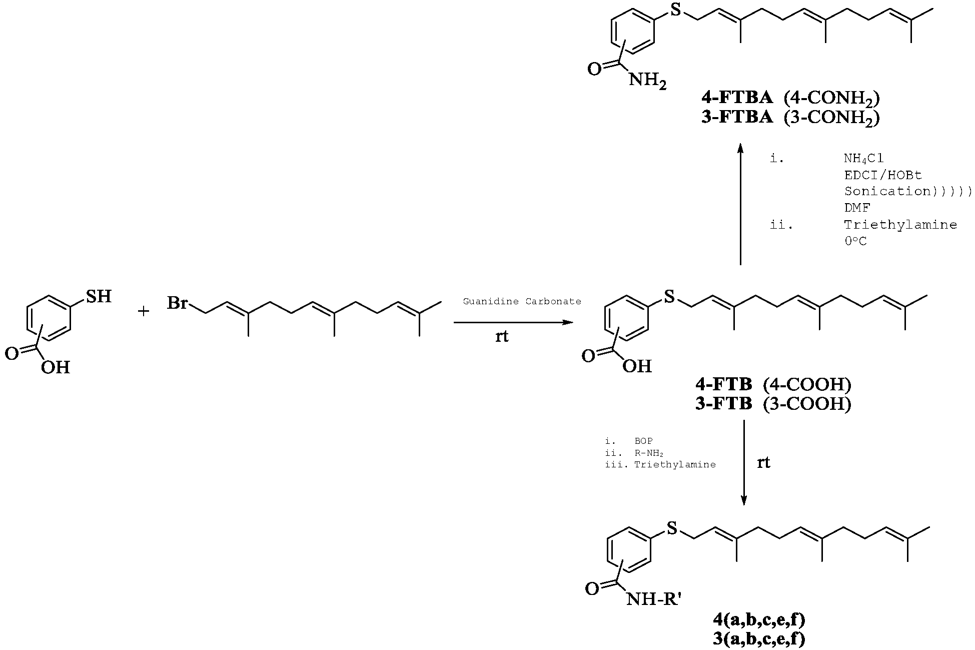

### General Synthesis Method for 3-Farnesylthiobenzamide (3-FTBA) and 4-Farnesylthiobenzamide (4-FTBA)

A mixture of 3-FTB (9.0 g) / 4-FTB (0.9 g) 1-ethyl-3(dimethylaminopropyl)carbodiimide hydrochloride (EDCI) (9.6 g), 1-hydroxybenzotriazole (HOBt) (6.75 g) and ammonium chloride (2.70 g) was subjected to ultrasonication for 5 minutes inside 150 ml of dimethylformamide and subsequently, the mixture was cooled under argon gas. Triethylamine (TEA) was added dropwise at 0°C and the mixture was further mixed for 2.5 hours inside an ice bath. Aqueous citric acid and aqueous sodium bicarbonate were added to the mixture and subsequently, extracted with ethyl acetate. The organic phase was dried with anhydrous magnesium sulfate and concentrated (Goldberg et al., 2009). The raw product was purified by using an n-hexane/ethyl acetate (1:1) solvent system via column chromatography.

### General Synthesis Method for 3/4-((2E,6E)-3,7,11-trimethyldodeca-2,6,10-trienylthio)benzamide Derivative Compounds (3(a, b, c, e, f); 4(a, b, c, e, f))

3-FTB (1.4 mmol) / 4-FTB (1.4 mmol) was mixed inside amine derivative compound (3.6 mmol) and triethylamine (0.7 ml) dichloromethane (50 ml) under argon gas and benzotriazole-1-yl-oxy-tri(dimethylamino)phosphonium hexafluorophosphate (BOP) (0.70 g) was added thereto. The mixture was stirred for 24 hours at room temperature and concentrated under vacuum (Goldberg et al., 2009). The raw product was purified via column chromatography by using suitable solvent systems.

### Synthesis and Analysis of Starting Materials (3-FTB, 4-FTB)

### 3-trans,trans-farnesylthiobenzoic acid (3-FTB; 3-((2E,6E)-3,7,11 trimethyldodeca-2,6,10-trienylthio)benzoic acid)

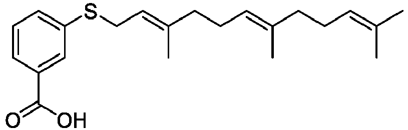

3-Mercaptobenzoic acid (0.9 g, 6 mmol), *trans,trans*-farnesylbromide (1.7 g, 6 mmol), and guanidine carbonate (1.5 g, 16.6 mmol) were mixed within 50 ml of acetone overnight at room temperature. Acetone was concentrated and chloroform was added along with 10 ml of 2N HCl. The mixture was washed with distilled water. The organic phase was separated and dried with magnesium sulfate and concentrated. The yellow-colored lipoid raw product was purified by using dichloromethane/ethyl acetate (5:1-2:1) solvent system via column chromatography (Tan, 1990; Parish, 1997). The yellowish-white solid substance obtained as a result was dried. The purity thereof was checked via Thin-Layer Chromatography (dichloromethane/ethyl acetate (5:1)).

Yield: 58.20% M.P.: 51°C. Elemental analysis, For C₂₂H₃₀O₂S; Calculated: C 73.70, H 8.43, N 8.94; Obtained: C 73.53, H 8.75, N 8.75. Mass Spectrum, ESI- MS (m/z %): 357 (M-H, 100). ¹H-NMR Spectrum, δ ppm (400 MHz, CDCl₃): 11.74 (br s, 1H, -COOH), 8.07 (s, 1H), 7.91 (d, 1H, *Jₒ*=8 Hz), 7.54 (d, 1H, *Jₒ*=7,6 Hz), 7.36 (t, 1H, *Jₒ*=8.0 Hz), 5.31 (t, 1H, *J*=7.2 Hz, SCH₂CH), 5.08 (m, 2H, 2 × CH₂CH=CCH₃), 3.61 (d, 2H, *J*=7.6 Hz, S-CH₂), 2.08-1.95 (m, 8H, 4CH₂), 1.68 (s, 3H, -CH₃), 1.63 (s, 3H, -CH₃), 1.60 (s, 3H, CH₃), 1.59 (s, 3H, CH₃).

### 4-trans,trans-farnesylthiobenzoic acid (4-FTB; 4-((2E,6E)-3,7,11-trimethyldodeca-2,6,10-trienylthio)benzoic acid)

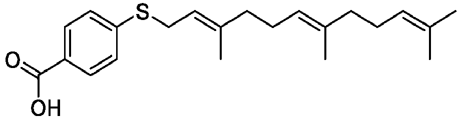

4-Mercaptobenzoic acid (0.9 g, 6 mmol), *trans,trans*-farnesylbromide (1.7 g, 6 mmol), and guanidine carbonate (1.5 g, 16.6 mmol) were mixed within 50 ml of acetone overnight at room temperature. Acetone was concentrated and chloroform was added along with 10 ml of 2N HCl. The mixture was washed with distilled water. The organic phase was separated and dried with magnesium sulfate and concentrated. The yellow-colored lipoid raw product was purified by using dichloromethane/ethyl acetate (5:1-2:1) solvent system via column chromatography. The yellowish-white solid substance obtained as a result was dried. The purity thereof was checked via Thin-Layer Chromatography (dichloromethane/ethyl acetate (5:1)).

Yield: 61.92%. M.P.: 78°C. Elemental Analysis, For C₂₂H₃₀O₂S; Calculated: C 73.70, H 8.43, N 8.94; Obtained: C 73.58, H 8.49, N 8.58. Mass Spectrum, ESI- (m/z %): 357 (M-H, 100). ¹H-NMR Spectrum, δ ppm (400 MHz, CDCl₃): 7.98 (d, 2H, *Jₒ*=8.4 Hz), 7.31 (d, 2H, *Jₒ*=8 Hz), 5.32 (t, 1H, *J*=7.6 Hz, SCH₂CH), 5.08 (m, 2H, 2 × CH₂CH=CCH₃), 3.64 (d, 2H, *J*=7.6 Hz, S-CH₂), 2.09-1.94 (m, 8H, 4CH₂), 1.71 (s, 3H, -CH₃), 1.68 (s, 3H, -CH₃), 1.60 (s, 3H, CH₃), 1.59 (s, 3H, CH₃).

### Synthesis of 3-Farnesylthiobenzamide (3-FTBA) and 4-Farnesylthiobenzamide (4-FTBA) Compounds

### 3-((2E,6E)-3,7,11-trimethyldodeca-2,6,10-trienylthio)benzamide (Compound 3-FTBA)

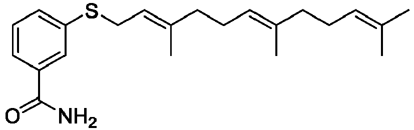

A mixture of 3-FTB (9.0 g), 1-ethyl-3(dimethylaminopropyl)carbodiimide hydrochloride (9.6 g), 1-hydroxybenzotriazole (6.75 g), and ammonium chloride (2.70 g) was subjected to ultrasonication for 5 minutes inside 150 ml of dimethylformamide, and subsequently, the mixture was cooled under argon gas. Triethylamine was added dropwise at 0°C and the mixture was further mixed for 2.5 hours inside an ice bath. Aqueous citric acid and aqueous sodium bicarbonate were added to the mixture and subsequently, extracted with ethyl acetate. The organic phase was dried with anhydrous magnesium sulfate and concentrated (Goldberg et al., 2009). The raw product was purified by using an n-hexane/ethyl acetate (1:1) solvent system via column chromatography. The bright yellow colored substance was manually dried.

The purity thereof was checked via Thin-Layer Chromatography (n-hexane/ethyl acetate (1:1)).

Yield: 44.18%. M.P.: 66°C. Elemental Analysis, For C₂₂H₃₁NOS-0.33H₂O; Calculated: C 72.69, H 8.78, N 3.85, S 8.82; Obtained: C 72.52, H 8.67, N 3.94, S 8.58. Mass Spectrum, ESI+ (m/z %): 358 (M+H, 26), 380 (M+Na, 80). ¹H-NMR Spectrum, δ ppm (400 MHz, CDCl₃): 7.76 (s, 1H), 7.57 (d, 1H, *Jₒ*=8 Hz), 7.46 (d, 1H, *Jₒ*=8 Hz), 7.33 (t, 1H, *Jₒ*=8 Hz), 5.98 (br d, 2H, NH₂), 5.29 (t, 1H, *J*=7.6 Hz, SCH₂CH), 5.07 (m, 2H, 2 × CH₂CH=CCH₃), 3.58 (d, 2H, *J*=8 Hz, S-CH₂), 2.04-1.93 (m, 8H, 4CH₂), 1.67 (s, 3H, -CH₃), 1.60 (s, 3H, -CH₃), 1.59 (s, 3H, CH₃), 1.57 (s, 3H, CH₃).

### 4-((2E,6E)-3,7,11-trimethyldodeca-2,6,10-trienylthio)benzamide (Compound 4-FTBA)

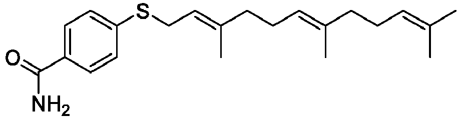

A mixture of 4-FTB (9.0 g), 1-ethyl-3(dimethylaminopropyl)carbodiimide hydrochloride (9.6 g), 1-hydroxybenzotriazole (6.75 g), and ammonium chloride (2.70 g) was subjected to ultrasonication for 5 minutes inside 150 ml of dimethylformamide, and subsequently, the mixture was cooled under argon gas. Triethylamine was added dropwise at 0°C and the mixture was further mixed for 2.5 hours inside an ice bath. Aqueous citric acid and aqueous sodium bicarbonate were added to the mixture and subsequently, extracted with ethyl acetate. The organic phase was dried with anhydrous magnesium sulfate and concentrated (Goldberg et al., 2009). The raw product was purified by using an n-hexane/ethyl acetate (1:1) solvent system via column chromatography. The bright yellow colored substance was dried. The purity thereof was checked via Thin-Layer Chromatography (n-hexane/ethyl acetate (1:1)).

Yield: 36.73%. M.P.: 90°C. Elemental Analysis, For C₂₂H₃₁NOS-0.2H₂O; Calculated: C 73.16, H 8.76, N 3.88, S 8.88; Obtained: C 73.21, H 8.45, N 3.78, S 8.50. Mass Spectrum, ESI+ (m/z %): 358 (M+H, 77), 380 (M+Na, 65). ¹H-NMR Spectrum, δ ppm (400 MHz, CDCl₃): 7.70 (d, 2H, *Jₒ*=8.4 Hz), 7.32 (d, 2H, Jₒ=8.8 Hz), 5.31 (t, 1H, *J*=7.6 Hz, SCH₂CH), 5.08 (m, 2H, 2 × CH₂CH=CCH₃), 3.62 (d, 2H, *J*=7.2 Hz, S-CH₂), 2.09-1.94 (m, 8H, 4CH₂), 1.68 (s, 6H, -CH₃), 1.60 (s, 3H, CH₃), 1.59 (s, 3H, CH₃).

### Synthesis of 3-((2E,6E)-3,7,11-trimethyldodeca-2,6,10-trienylthio)benzamide Derivative Compounds and Clarification of Structures Thereof

### N-(2-(1H-Indole-3-yl)ethyl)-3-((2E,6E)-3,7,11-trimethyldodeca-2,6,10-trienylthio)benzamide (Compound 3a)

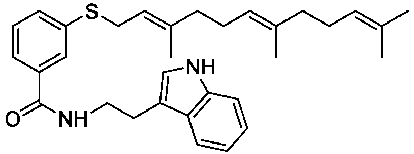

3-FTB (0.5 g, 1.4 mmol), 2-(1*H*-indole-3-yl)ethanamine (0.58 g, 3.6 mmol) and triethylamine (0.7 ml) were mixed within dichloromethane (50 ml) under argon gas and benzotriazole-1-yl-oxy-tri(dimethylamino)phosphonium hexafluorophosphate (BOP) (0.7 g) was added thereto. The mixture was stirred for 24 hours at room temperature and concentrated under vacuum (Goldberg et al., 2009). The dark yellow colored lipoid raw product was purified by using dichloromethane/ethyl acetate (5:1-2:1) solvent system via column chromatography. The obtained yellow colored substance was dried. The purity thereof was checked via Thin-Layer Chromatography (dichloromethane/ethyl acetate (5:1)).

Yield: 76.70%. M.P.: 78°C. Elemental Analysis, For C₃₂H₄₀N₂OS; Calculated: C 76.76, H 8.05, N 5.59, S 6.40; Obtained: C 76.58, H 8.08, N 5.80, S 6.39. Mass Spectrum, ESI+ (m/z %): 501 (M+H, 42), 523 (M+Na, 100). ¹H-NMR Spectrum, δ ppm (400 MHz, CDCl₃): 8.09 (br s, 1H, -NH), 7.62 (s, 1H), 7.63 (d, 1H, *Jₒ*=8.4 Hz), 7.42-7.36 (m, 3H), 7.27-7.19 (m, 2H), 7.12 (t, 1H, *Jₒ*=7.2 Hz), 7.05 (d, 1H, *J*=1.6 Hz), 6.16 (br t, 1H, CO-NH), 5.26 (t, 1H, *J*=7.2 Hz, SCH₂CH), 5.05 (m, 2H, 2 x CH₂CH=CCH₃), 3.78 (q, 2H, N-CH₂), 3.53 (d, 2H, *J*=7.2 Hz, S-CH₂), 3.08 (t, 2H, *J*=6.4 Hz, Ar-CH₂), 2.05-1.93 (m, 8H, 4CH₂), 1.66 (s, 3H, -CH₃), 1.58 (s, 6H, -CH₃), 1.57 (s, 3H, -CH₃).

### N-(2-(5-Methoxy-1H-indole-3-yl)ethyl)-3-((2E,6E)-3,7,11-trimethyldodeca-2,6,10-trienylthio)benzamide (Compound 3b)

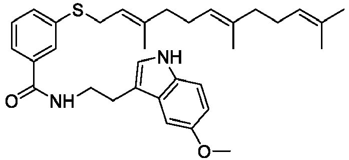

3-FTB (0.5 g, 1.4 mmol), 2-(5-methoxy-1H-indole-3-yl)ethanamine (0.68 g, 3.6 mmol) and triethylamine (0.7 ml) were mixed inside dichloromethane (50 ml) under argon gas and benzotriazole-1-yl-oxy-tri(dimethylamino)phosphonium hexafluorophosphate (BOP) (0.7 g) was added thereto. The mixture was stirred for 24 hours at room temperature and concentrated under vacuum (Goldberg et al., 2009). The dark yellow colored lipoid raw product was purified by using a dichloromethane/ethyl acetate (5:1) solvent system via column chromatography. The obtained yellow-colored lipoid substance was dried. The purity thereof was checked via Thin-Layer Chromatography (dichloromethane/ethyl acetate (5:1)).

Yield: 81.75%. Elemental Analysis, For C₃₃H₄₂N₂O₂S; Calculated C 74.68, H 7.98, N 5.28, S 6.04; Obtained C 74.66, H 8.12, N 5.48, S 6.04. Mass Spectrum, ESI+ (m/z %): 531 (M+H, 92), 553 (M+Na, 100). ¹H-NMR Spectrum, δ ppm (400 MHz, CDCl₃): 8.14 (br s, 1H, -NH), 7.64 (s, 1H), 7.41 (d, 1H, *Jₒ=9.2* Hz), 7.39 (d, 1H, *Jₒ*=9.6 Hz), 7.25 (d, 1H, *Jₒ=9.2* Hz), 7.24 (t, 1H, *Jₒ*=8.0 Hz), 7.03 (d, 1H, *Jₘ=2.0* Hz), 7.02 (s, 1H), 6.86 (dd, 1H, *Jₒ*=8.8 Hz, *Jₘ*=2.4 Hz), 6.25 (br t, 1H, CO-NH), 5.26 (t, 1H, J=7.6 Hz, SCH₂CH), 5.07 (m, 2H, 2 × CH₂CH=CCH₃), 3.78 (s, 3H, -OCH₃), 3.76 (q, 2H, N-CH₂), 3.53 (d, 2H, *J*=7.6 Hz, S-CH₂), 3.04 (t, 2H, *J*=6.8 Hz, Ar-CH₂), 2.07-1.93 (m, 8H, 4CH₂), 1.67 (s, 3H, -CH₃), 1.59 (s, 6H, -CH₃), 1.58 (s, 3H, - CH₃).

### N-(2,2,6,6-Tetramethylpiperidine-1-oxyl-4-yl)-3-((2E,6E)-3,7,11-trimethyldodeca-2,6,10-trienylthio)benzamide (Compound 3c)

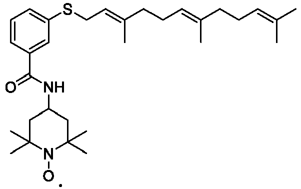

3-FTB (0.5 g, 1.4 mmol), 4-amino-2,2,6,6-tetramethylpiperidine-1-oxyl (4-amino-TEMPO) (0.62 g, 3.6 mmol) and triethylamine (0.7 ml) were mixed inside dichloromethane (50 ml) under argon gas and benzotriazole-1-yl-oxy-tri(dimethylamino)phosphonium hexafluorophosphate (BOP) (0.7 g) was added thereto. The mixture was stirred for 24 hours at room temperature and concentrated under vacuum (Goldberg et al., 2009). The orange-colored lipoid raw product was purified initially by using dichloromethane/ethyl acetate (5:1-2:1), and subsequently, by using hexane/ethyl acetate (1:1) solvent systems via column chromatography. The obtained orange-colored lipoid substance was dried. The purity thereof was checked via Thin-Layer Chromatography (dichloromethane/ethyl acetate (5:1)).

Yield: 42.10%. Elemental Analysis, For C₃₁H₄₇N₂O₂S^{•}; Calculated: C 72.75, H 9.26, N 5.47, S 6.27; Obtained C 72.82, H 9.09, N 5.47, S 6.11.

### N-(1H-Benzo[d]imidazole-2-yl)-3-((2E,6E)-3,7,11-trimethyldodeca-2,6,10 trienylthio)benzamide (Compound 3e)

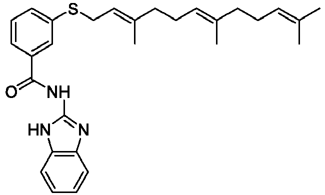

3-FTB (0.5 g, 1.4 mmol), 2-aminobenzimidazole (0.48 g, 3.6 mmol) and triethylamine (0.7 ml) were mixed inside dichloromethane (50 ml) under argon gas and benzotriazole-1-yl-oxy-tri(dimethylamino)phosphonium hexafluorophosphate (BOP) (0.7 g) was added thereto. The mixture was stirred for 24 hours at room temperature and concentrated under vacuum (Goldberg et al., 2009). The bright yellow-colored raw product was purified by using a dichloromethane/ethyl acetate (5:1) solvent system via column chromatography. The obtained white-colored substance was dried. The purity thereof was checked via Thin-Layer Chromatography (dichloromethane/ethyl acetate (5:1)).

Yield: 72.28%. M.P.: 74°C. Elemental Analysis, For C₂₉H₃₅N₃OS; Calculated: C 73.53, H 7.45, N 8.87, S 6.77; Obtained: C 73.67, H 7.55, N 8.78, S 6.77. Mass Spectrum, ESI+ (m/z %): 474 (M+H, 100). ¹H-NMR Spectrum, δ ppm (400 MHz, CDCl₃): 8.01 (s, 1H), 7.88 (d, 1H, *Jₒ*=8.0 Hz), 7.48 (d, 1H, *Jₒ*=8.0 Hz), 7.27 (t, 2H, *Jₒ*=8.0 Hz), 7.12 (m, 3H), 5.23 (t, 1H, SCH₂CH), 5.05 (m, 2H, 2 × CH₂CH=CCH₃), 3.47 (d, 2H, *J*=7.6 Hz, S-CH₂), 2.08-1.92 (m, 8H, 4CH₂), 1.66 (s, 3H, -CH₃), 1.60 (s, 3H, -CH₃), 1.58 (s, 3H, -CH₃), 1.56 (s, 3H, -CH₃).

### N-(Benzo[d]thiazole-2-yl)-3-((2E,6E)-3,7,11-trimethyldodeca-2,6,10-trienylthio)benzamide (Compound 3f)

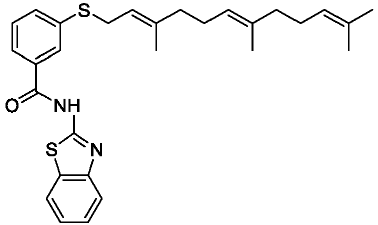

3-FTB (0.5 g, 1.4 mmol), 2-aminobenzothiazole (0.54 g, 3.6 mmol) and triethylamine (0.7 ml) were mixed inside dichloromethane (50 ml) under argon gas and benzotriazole-1-yl-oxy-tri(dimethylamino)phosphonium hexafluorophosphate (BOP) (0.7 g) was added thereto. The mixture was stirred for 24 hours at room temperature and concentrated under vacuum (Goldberg et al., 2009). The bright yellow-colored raw product was purified by using dichloromethane/ethyl acetate (5:1-2:1) solvent system via column chromatography. The obtained white-colored substance was dried. The purity thereof was checked via Thin-Layer Chromatography (dichloromethane/ethyl acetate (5:1)).

Yield: 76.92%. M.P.: 75°C. Elemental Analysis, For C₂₉H₃₄N₂OS₂; Calculated: C 70.98, H 6.98, N 5.71, S 13.07; Obtained C 70.68, H 7.11, N 5.83, S 12.72. Mass Spectrum, ESI+ (m/z %): 491 (M+H, 100). ¹H-NMR Spectrum, δ ppm (400 MHz, CDCl₃): 7.93 (s, 1H), 7.84 (m, 1H), 7.74 (d, 1H, *Jₒ*=8.0 Hz), 7.46 (d, 1H, *Jₒ*=8.0 Hz), 7.25-7.32 (m, 4H), 5.24 (t, 1H, *J*=8.0 Hz, SCH₂CH), 5.06 (m, 2H, 2 x CH₂CH=CCH₃), 3.48 (d, 2H, *J*=8.0 Hz, S-CH₂), 2.06-1.92 (m, 8H, 4CH₂), 1.66 (s, 3H, -CH₃), 1.62 (s, 3H, -CH₃), 1.58 (s, 3H, -CH₃), 1.57 (s, 3H, -CH₃).

### Synthesis of 4-((2E,6E)-3,7,11-trimethyldodeca-2,6,10-trienylthio)benzamide Derivative Compounds and Clarification of Structures Thereof

### N-(2-(1H-Indole-3-yl)ethyl)-4-((2E,6E)-3,7,11-trimethyldodeca-2,6,10-trienylthio)benzamide (Compound 4a)

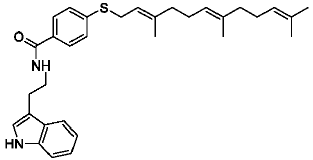

4-FTB (0.5 g, 1.4 mmol), 2-(1*H*-indole-3-yl)ethanamine (0.58 g, 3.6 mmol) and triethylamine (0.7 ml) were mixed inside dichloromethane (50 ml) under argon gas and benzotriazole-1-yl-oxy-tri(dimethylamino)phosphonium hexafluorophosphate (BOP) (0.7 g) was added thereto. The mixture was stirred for 24 hours at room temperature and concentrated under vacuum (Goldberg et al., 2009). The yellow-colored lipoid raw product was purified by using dichloromethane/ethyl acetate (5:1-2:1) solvent system via column chromatography. The obtained white-colored substance was dried. The purity thereof was checked via Thin-Layer Chromatography (dichloromethane/ethyl acetate (5:1)).

Yield: 37.20%. M.P.: 85°C. Elemental Analysis, For C₃₂H₄₀N₂OS; Calculated: C 76.76, H 8.05, N 5.59, S 6.40; Obtained: C 77.04, H 8.27, N 5.77, S 6.43. Mass Spectrum, ESI+ (m/z %): 501 (M+H, 100). ¹H-NMR Spectrum, δ ppm (400 MHz, CDCl₃): 8.13 (br s, 1H, -NH), 7.64 (d, 1H, *Jₒ*=7.6 Hz), 7.55 (d, 2H, *Jₒ*=8.1 Hz), 7.38 (d, 1H, *Jₒ*=8.1 Hz), 7.25 (d, 2H, *Jₒ*=8.8 Hz), 7.21 (t, 1H, *Jₒ*=8.1 Hz, *Jₘ*=1.2 Hz), 7.13 (t, 1H, *Jₒ*=7.6 Hz, *Jₘ*=1.2 Hz), 7.06 (d, 1H, *J*=2.4 Hz), 6.19 (br t, 1H, CO-NH), 5.29 (t, 1H, *J*=7.2 Hz, SCH₂CH), 5.07 (m, 2H, 2 x CH₂CH=CCH₃), 3.79 (q, 2H, N-CH₂), 3.59 (d, 2H, *J*=7.2 Hz, S-CH₂), 3.09 (t, 2H, *J*=6.8 Hz, Ar-CH₂), 2.08-1.94 (m, 8H, 4CH₂), 1.68 (s, 3H, -CH₃), 1.66 (s, 3H, -CH₃), 1.60 (s, 3H, -CH₃), 1.58 (s, 3H, -CH₃).

### N-(2-(5-Methoxy-1H-indole-3-yl)ethyl)-4-((2E,6E)-3,7,11-trimethyldodeca-2,6,10-trienylamino)benzamide (Compound 4b)

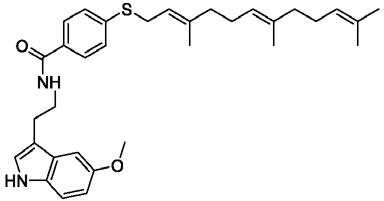

4-FTB (0.25 g, 0.7 mmol), 2-(5-methoxy-1H-indole-3-yl)ethanamine (0.36 g, 1.9 mmol) and triethylamine (0.3 ml) were mixed inside dichloromethane (50 ml) under argon gas and benzotriazole-1-yl-oxy-tri(dimethylamino)phosphonium hexafluorophosphate (BOP) (0.36 g) was added thereto. The mixture was stirred for 24 hours at room temperature and concentrated under vacuum. The yellow-colored raw product was purified by using dichloromethane/ethyl acetate (5:1-2:1) solvent system via column chromatography. The obtained bright yellow colored substance was dried. The purity thereof was checked via Thin-Layer Chromatography (dichloromethane/ethyl acetate (5:1)).

Yield: 64.86%. M.P. :72°C. Elemental Analysis, For C₃₃H₄₂N₂O₂S; Calculated: C 74.68, H 7.98, N 5.28, S 6.04; Obtained: C 74.92, H 8.26, N 5.43, S 6.02. Mass Spectrum, ESI+ (m/z %): 531 (M+H, 100). ¹H-NMR Spectrum, δ ppm (400 MHz, CDCl₃): 8.04 (br s, 1H, -NH), 7.55 (d, 2H, *Jₒ*=8.1 Hz), 7.26 (m, 3H), 7.04 (d, 1H, *Jₘ*=2.8 Hz), 7.03 (s, 1H), 6.87 (dd, 1H, *Jₒ=9.2* Hz, Jₘ=2.8 Hz), 6.20 (br s, 1H, CO-NH), 5.29 (t, 1H, *J*=8.0 Hz, SCH₂CH), 5.08 (m, 2H, 2 x CH₂CH=CCH₃), 3.78 (s, 3H, -OCH₃), 3.77 (q, 2H, N-CH₂), 3.58 (d, 2H, *J*=7,2 Hz, S-CH₂), 3.05 (t, 2H, *J*=6.8 Hz, Ar-CH₂), 2.08-1.93 (m, 8H, 4CH₂), 1.67 (s, 3H, -CH₃), 1.65 (s, 3H, -CH₃), 1.59 (s, 3H, -CH₃), 1.58 (s, 3H, -CH₃).

### N-(2,2,6,6-Tetramethylpiperidine-1-oxyl-4-yl)-4-((2E,6E)-3,7,11-trimethyldodeca-2,6,10-trienylthio)benzamide (Compound 4c)

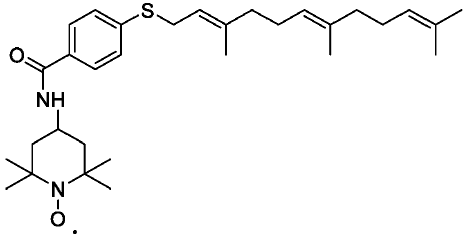

4-FTB (0.5 g, 1.4 mmol), 4-amino-2,2,6,6-tetramethylpiperidine-1-oxyl (4-amino-TEMPO) (0.62 g, 3.6 mmol) and triethylamine (0.7 ml) were mixed inside dichloromethane (50 ml) under argon gas and benzotriazole-1-yl-oxy-tri(dimethylamino)phosphonium hexafluorophosphate (BOP) (0.7 g) was added thereto. The mixture was stirred for 24 hours at room temperature and concentrated under vacuum (Goldberg et al., 2009). Orange colored lipoid raw product was purified by using dichloromethane/ethyl acetate (5:1) solvent system via column chromatography. The obtained orange-colored lipoid substance was dried. The purity thereof was checked via Thin-Layer Chromatography (dichloromethane/ethyl acetate (5:1)).

Yield: 31.58%. Elemental Analysis, For C₃₁H₄₇N₂O₂S^{•}; Calculated: C 72.75, H 9.26, N 5.47, S 6.27; Obtained: C 72.92, H 9.44, N 5.61, S 6.24. Mass Spectrum, ESI+ (m/z %): 512 (M+H, 100). ¹H-NMR Spectrum, δ ppm (400 MHz, CDCl₃): 7.98 (d, 2H, *Jₒ*=8.4 Hz), 7.31 (d, 2H, *Jₒ*=8.0 Hz), 5.32 (t, 1H, *J*=7.6 Hz, SCH₂CH), 5.08 (m, 2H, 2 × CH₂CH=CCH₃), 3.64 (d, 2H, *J*=7.6 Hz, S-CH₂), 2.09-1.94 (m, 8H, 4CH₂), 1.71 (s, 3H, -CH₃), 1.68 (s, 3H, -CH₃), 1.60 (s, 3H, CH₃), 1.59 (s, 3H, CH₃).

### N-(1H-Benzo[d]imidazole-2-yl)-4-((2E,6E)-3,7,11-trimethyldodeca-2,6,10-trienylthio)benzamide (Compound 4e)

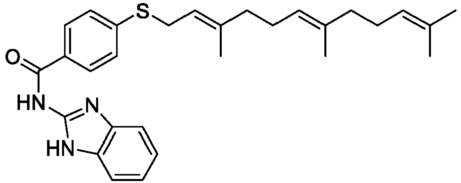

4-FTB (0.5 g, 1.4 mmol), 2-aminobenzimidazole (0.48 g, 3.6 mmol) and triethylamine (0.7 ml) were mixed inside dichloromethane (50 ml) under argon gas and benzotriazole-1-yl-oxy-tri(dimethylamino)phosphonium hexafluorophosphate (BOP) (0.7 g) was added thereto. The mixture was stirred for 24 hours at room temperature and concentrated under vacuum (Goldberg et al., 2009). The bright yellow-colored raw product was purified by using dichloromethane/ethyl acetate (5:1-2:1) solvent system via column chromatography. The obtained white-colored substance was dried. The purity thereof was checked via Thin-Layer Chromatography (dichloromethane/ethyl acetate (5:1)).

Yield: 33.33% M.P.: 136°C. Elemental Analysis, For C₂₉H₃₅N₃OS; Calculated: C 73.53, H 7.45, N 8.87, S 6.77; Obtained: C 73.54, H 7.62, N 8.83, S 6.73. Mass Spectrum, ESI+ (m/z %): 474 (M+H, 100). ¹H-NMR Spectrum, δ ppm (400 MHZ, CDCl₃): 7.99 (d, 2H, *Jₒ*=8.4 Hz), 7.24 (m, 3H), 7.14 (m, 3H), 5.30 (t, 1H, *J*=7.2 Hz, SCH₂CH), 5.07 (m, 2H, 2 x CH₂CH=CCH₃), 3.58 (d, 2H, *J*=7.2 Hz, S-CH₂), 2.09-1.93 (m, 8H, 4CH₂), 1.68 (s, 3H, -CH₃), 1.66 (s, 3H, - CH₃), 1.58 (s, 6H, -CH₃).

### N-(Benzo[d]thiazole-2-yl)-4-((2E,6E)-3,7,11-trimethyldodeca-2,6,10-trienylthio)benzamide (Compound 4f)

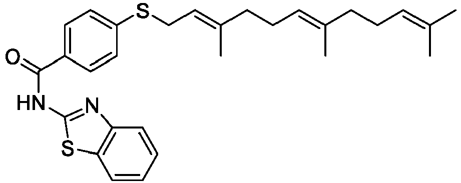

4-FTB (0.5 g, 1.4 mmol), 2-aminobenzothiazole (0.54 g, 3.6 mmol) and triethylamine (0.7 ml) were mixed inside dichloromethane (50 ml) under argon gas and benzotriazole-1-yl-oxy-tri(dimethylamino)phosphonium hexafluorophosphate (BOP) (0.7 g) was added thereto. The mixture was stirred for 24 hours at room temperature and concentrated under vacuum (Goldberg et al., 2009). The bright yellow-colored raw product was purified by using dichloromethane/ethyl acetate (5:1-2:1) solvent system via column chromatography. The obtained white-colored substance was dried. The purity thereof was checked via Thin-Layer Chromatography (dichloromethane/ethyl acetate (5:1)).

Yield: 67.40% M.P.: 74°C. Elemental Analysis, For C₂₉H₃₄N₂OS₂-0.05H₂O; Calculated: C 70.85, H 6.99, N 5.70, S 13.05; Obtained: C 70.51, H 7.04, N 5.90, S 12.66. Mass Spectrum, ESI+ (m/z %): 491 (M+H, 100). ¹H-NMR Spectrum, δ ppm (400 MHZ, CDCl₃): 7.89-7.83 (m, 3H), 7.44 (dd, 1H, *Jₒ*=7.2 Hz, *Jₘ*=1.2 Hz), 7.37-7.20 (m, 4H), 5.28 (m, 1H, SCH₂CH), 5.07 (m, 2H, 2 × CH₂CH=CCH₃), 3.58 (m, 2H, S-CH₂), 2.09-1.93 (m, 8H, 4CH₂), 1.69 (s, 3H, -CH₃), 1.67 (s, 3H, -CH₃), 1.59 (s, 6H, -CH₃).

Based on the anticancer effects of the FTS compound, the present invention comprises the synthesis of 14 substances, 12 of which are entirely original. Initially, the phenyl ring in the FTS compound and the farnesylthio- group attached to the ring were preserved, and carboxylic acid-bound compounds (3-FTB, 4-FTB) were synthesized from the meta and para positions based on the farnesylthio- group attached to the phenyl ring. Subsequently, non-substituted amide (3-FTBA, 4-FTBA) and substituted amide derivatives (3 (a, b, c, e, f), 4 (a, b, c, e, f)) were synthesized over the 3-FTB and 4-FTB compounds.

Synthesized compounds were purified via column chromatography by using suitable solvent systems. Purities of compounds were checked via TLC and melting points of the ones in solid form were determined. Accordingly, expected chemical structures of compounds were substantiated through the results of elemental analysis, mass (ESI), as well as ¹H-NMR spectral results.

While the structures of compounds 3c and 4c carrying tempamine residue were clarified, protons and cleavages thereof were not clear in the ¹H-NMR spectrum due to their free radical characteristic. Although quenching the paramagnetic nitroxide radical (-N-O.) in the NMR tube, and thereby reducing it to the corresponding hydroxylamine (-N-OH) (Franchi et al., 2008) was considered while the 1H-NMR spectrum was being taken, the 1H-NMR spectrum was not obtained due to the resolution problem of the compound obtained as a result of the reduction.

## Claims

1. A benzamide derivative compound for use in cancer treatment, **characterized by** being one or more than one of the ones defined in the following group:
3-((2*E*,6*E*)-3,7,11-trimethyldodeca-2,6,10-trienylthio)benzamide,
4-((2*E*,6*E*)-3,7,11-trimethyldodeca-2,6,10-trienylthio)benzamide,
*N*-(2-(1*H*-Indole-3-yl)ethyl)-3-((2*E*,6*E*)-3,7,11-trimethyldodeca-2,6,10-trienylthio)benzamide,
*N*-(2-(5-Methoxy-1*H*-indole-3-yl)ethyl)-3-((2*E*,6*E*)-3,7,11-trimethyldodeca-2,6,10-trienylthio)benzamide,
*N*-(2,2,6,6-Tetramethylpiperidine-1-oxyl-4-yl)-3-((2*E*,6*E*)-3,7,11-trimethyldodeca-2,6,10-trienylthio)benzamide,
*N*-(1H-Benzo[d]imidazole-2-yl)-3-((2*E*,6*E*)-3,7,11-trimethyldodeca-2,6,10-trienylthio)benzamide,
*N*-(Benzo[d]thiazole-2-yl)-3-((2*E*,6*E*)-3,7,11-trimethyldodeca-2,6,10-trienylthio)benzamide,
*N*-(2-(1*H*-Indole-3-yl)ethyl)-4-((2*E*,6*E*)-3,7,11-trimethyldodeca-2,6,10-trienylthio)benzamide,
*N*-(2-(5-Methoxy-1*H*-indole-3-yl)ethyl)-4-((2*E*,6*E*)-3,7,11-trimethyldodeca-2,6,10-trienylthio)benzamide,
*N*-(2,2,6,6-Tetramethylpiperidine-1-oxyl-4-yl)-4-((2*E*,6*E*)-3,7,11-trimethyldodeca-2,6,10-trienylthio)benzamide,
*N*-(1*H*-Benzo[d]imidazole-2-yl)-4-((2*E*,6*E*)-3,7,11-trimethyldodeca-2,6,10-trienylthio)benzamide,
*N*-(Benzo[d]thiazole-2-yl)-4-((2*E*,6*E*)-3,7,11-trimethyldodeca-2,6,10-trienylthio)benzamide.

## Patentansprüche

1. Benzamid-Derivatverbindung zur Verwendung in der Krebsbehandlung, **dadurch gekennzeichnet, dass** sie eine oder mehrere von denjenigen ist, die in der folgenden Gruppe definiert werden:
3-((*2E,6E*)-3,7,11-trimethyldodeca-2,6,10-trienylthio)benzamid,
*4-((2E, 6E)-3,7,1 1* -trimethyldodeca-2,6,10-trienylthio)benzamid,
*N*-(2-(1*H*-Indol-3-yl)ethyl)-3-((*2E*,*6E*)-3,7,11-trimethyldodeca-2,6,10-trienylthio)benzamid,
*N*-(2-(5-Methoxy-1*H*indol-3-yl)ethyl)-3-((2Ä, 6E)-3,1,11 -trimethyldodeca-2,6,10-trienylthio)benzamid,
*N*-(2,2,6,6-Tetramethylpiperidin-1 -oxyl-4-yl)-3-((2E, 6E)-3,1,11 -trimethyldodeca-2,6,10-trienylthio)benzamid,
*N*-(1*H*-Benzo[d]imidazol-2-yl)-3-((*2E*,*6E*)-3,7,11-trimethyldodeca-2,6,10-trienylthio)benzamid,
*N-*(Benzo[d]thiazol-2-yl)-3-((*2E*,*6E*)-3,7,11-trimethyldodeca-2,6,10-trienylthio)benzamid,
*N*-(2-(1*H*-Indol-3-yl)ethyl)-4-((*2E*,*6E*)-3,7,11-trimethyldodeca-2,6,10-trienylthio)benzamid,
*N-*(2-(5-Methoxy-1*H*-indol-3-yl)ethyl)-4-((*2E*,*6E*)-3,7,11-trimethyldodeca-2,6,10-trienylthio)benzamid,
*N*-(2,2,6,6-Tetramethylpiperidin-1-oxyl-4-yl)-4-((*2E*,*6E*)-3,7,11-trimethyldodeca-2,6,10-trienylthio)benzamid,
*N*-(1*H*-Benzo[d]imidazol-2-yl)-4-((*2E*,*6E*)-3,7,11-trimethyldodeca-2,6,10-trienylthio)benzamid,
*N*-(Benzo[d]thiazol-2-yl)-4-((*2E*,*6E*)-3,1,11-trimethyldodeca-2,6,10-trienylthio)benzamid.

## Revendications

1. Composé dérivé du benzamide pour utilisation dans le traitement du cancer, **caractérisé par le fait qu'**il est l'un ou plus d'un de ceux définis dans le groupe suivant:
*3-((2E,6E*)-3,7,11-triméthyldodéca-2,6,10-trienylthio)benzamide,
*4-((2E,6E*)-3,7,11-triméthyldodéca-2,6,10-trienylthio)benzamide,
*N*-(2-(1*H*-Indole-3-yl)éthyl)-3-((*2E*,*6E*)-3,*7*,11-triméthyldodéca-2,6,10-trienylthio)benzamide,
*N*-(2-(5-Méthoxy-1*H*-indole-3-yl)éthyl)-3-((*2E*,*6E*)-3,7,11-triméthyldodéca-2,6,10-trienylthio)benzamide,
*N*-(2,2,6,6-Tétraméthylpipéridine-1-oxyl-4-yl)-3-((*2E*,*6E*)-3,7,11-triméthyldodéca-2,6,10-trienylthio)benzamide,
*N*-(1*H*-Benzo[d]imidazole-2-yl)-3-((*2E*,*6E*)-3,7,11-triméthyldodéca-2,6,10-trienylthio)benzamide,
*N-*(Benzo[d]thiazole-2-yl)-3-((*2E*,*6E*)-3,7,11-triméthyldodéca-2,6,10-trienylthio)benzamide,
*N*-(2-(1*H*-Indole-3-yl)éthyl)-4-((*2E*,*6E*)-3,7,11-triméthyldodéca-2,6,10-trienylthio)benzamide,
*N*-(2-(5-Méthoxy-1*H*-indole-3-yl)éthyl)-4-((*2E*,*6E*)-3,7,11-triméthyldodéca-2,6,10-trienylthio)benzamide,
*N*-(2,2,6,6-Tétraméthylpipéridine-1-oxyl-4-yl)-4-((2*E,6E*)-3,7,11-triméthyldodéca-2,6,10-trienylthio)benzamide,
*N*-(1*H*-Benzo[d]imidazole-2-yl)-4-((*2E*,*6E*)-3,7,11-triméthyldodéca-2,6,10-trienylthio)benzamide,
*N*-(Benzo[d]thiazole-2-yl)-4-((*2E*,*6E*)-3,7,11-triméthyldodéca-2,6,10-trienylthio)benzamide.
